# EUROPEAN PATENT APPLICATION

(11) **EP 2 347 786 A1**
(43) Date of publication of application: **27.07.2011**
(21) Application number: 10196826.1
(22) Date of filing: 23.12.2010
(51) Int. Cl.: A61M 25/09

(54) **Medical guidewire**

(30) Priority: 21.01.2010 JP 2010010589
(71) Applicant: ASAHI INTECC CO., LTD., Nagoya-shi, Aichi 463-0024 (JP)
(72) Inventor: Maki, Hideaki, Aichi 463-0024 (JP); Goto, Shinichi, Aichi 463-0024 (JP); Kato, Tadakazu, Aichi 463-0024 (JP)
(74) Representative: Zimmermann, Tankred Klaus

(57) **Abstract**

There is provided a medical guidewire (1), which is capable of reducing the sliding resistance generated in the case of pulling the medical guidewire inside a catheter, a tubular organ or an intracorporeal tissue, and thus has favorable manipulability for a physician who performs an operation. An area between a proximal end portion of a coiled body (5) and a core shaft (3) is formed in streamlined shape. It is thus possible to reduce the sliding resistance generated in the case of pulling the medical guidewire inside the catheter, the tubular organ or the intracorporeal tissue. Hence it is possible to provide the medical guidewire with favorable manipulability for the physician who performs the operation. It is to be noted that the streamlined shape is preferably formed by use of a brazing material that joins the proximal end portion of the coiled body with the core shaft.

## Description

### Cross Reference to related Application

This application is based of Japanese Patent Application No. 2010-010589 filed with the Japan patent Office on January 21,2010, the entire content of which is hereby incorporated by reference.

### Technical Field

The present invention relates to a medical guidewire.

### Background Art

There have conventionally been proposed a variety of medical guidewires for guiding a medical equipment such as a catheter which is used by being inserted into tubular organs such as blood vessels, digestive tracts and ureters, and intracorporeal tissues.

For example, a medical guidewire described in Patent Literature 1 (Japanese Patent Application Laid-Open No. 2008-307367) is made up of a wire body, a coil, a resin coating layer coated on this coil, and an annular member. The wire body has a certain diameter in a portion closer to the rear end than a tip portion, which is formed in tapered shape. The coil is a spiral coil that is wound around the tip portion of the wire body. The annular member is filled in a stepped space between a proximal end portion of the resin coating layer and the wire body.

Further, a hydrophilic lubricating layer is formed on the outer surface of the resin coating layer and the outer surface of the annular member in the medical guidewire described in Patent Literature 1.

It is described in Patent Literature 1 that in the case of using the medical guidewire and medical equipment in combination, the medical guidewire is prevented from getting caught in the medical equipment according to the invention described in this literature.

### Summary of Invention

In the medical guidewire described in Patent Literature 1, the annular member is filled in the stepped space between the proximal end portion of the resin coating layer and the wire body. However, a depressed portion with a corner is formed between the wired body and the annular member. There has thus been a problem in that the sliding resistance that occurs at the time of manipulating the medical guidewire increases due to this depressed portion.

Further, since the depressed portion of the medical guidewire described in Patent Literature 1 has the corner, there has been a problem in that a bodily fluid such as blood may be accumulated in this depressed portion.

Moreover, when the guiding catheter is curved, the sliding resistance between the medical guidewire and the guiding catheter also increases. It has thus been necessary to reduce such sliding resistance.

The present invention has been made in view of such circumstances. A first object of the present invention is to reduce the sliding resistance at the time of manipulation, especially the sliding resistance generated in the case of curving a guiding catheter. Further, a second object of the present invention is to provide a medical guidewire which does not inhibit the flow of a bodily fluid such as blood at the time of manipulation.

A medical guidewire of the present invention is a medical guidewire including a core shaft and a coiled body that covers at least a tip portion of the core shaft, wherein especially an area between a proximal end portion of the coiled body and the core shaft is formed in streamlined shape.

### Brief Description of Drawings

The foregoing and other objects, features, aspects and advantages of the invention will become more apparent from the following detailed description when taken in conjunction with the accompanying drawings.
Fig. 1

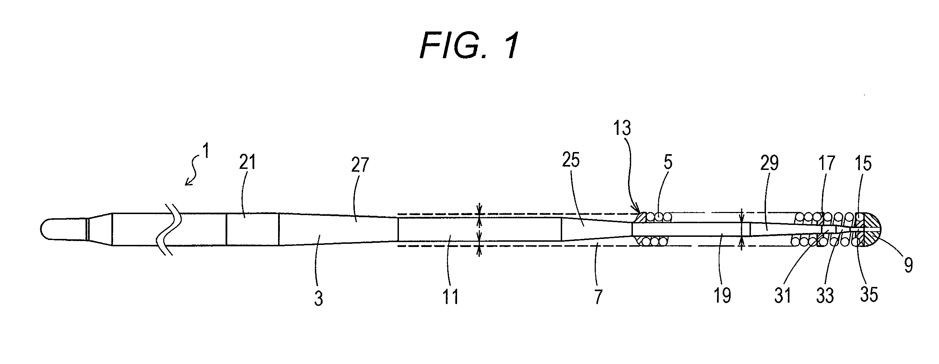

   Fig. 1 illustrates an overall view of a medical guidewire according to a first embodiment of the present invention.
Fig. 2

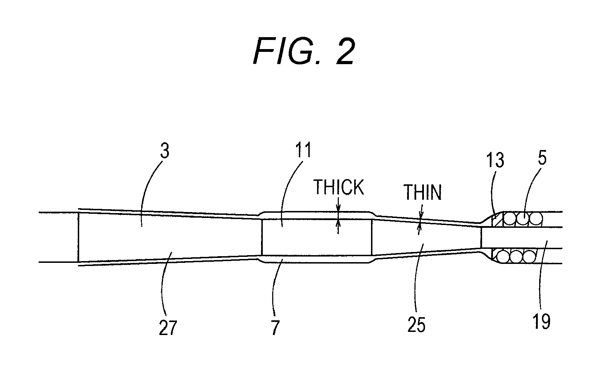

   Fig. 2 illustrates a partially enlarged view of a second cylindrical part of a core shaft in the first embodiment.
Fig. 3

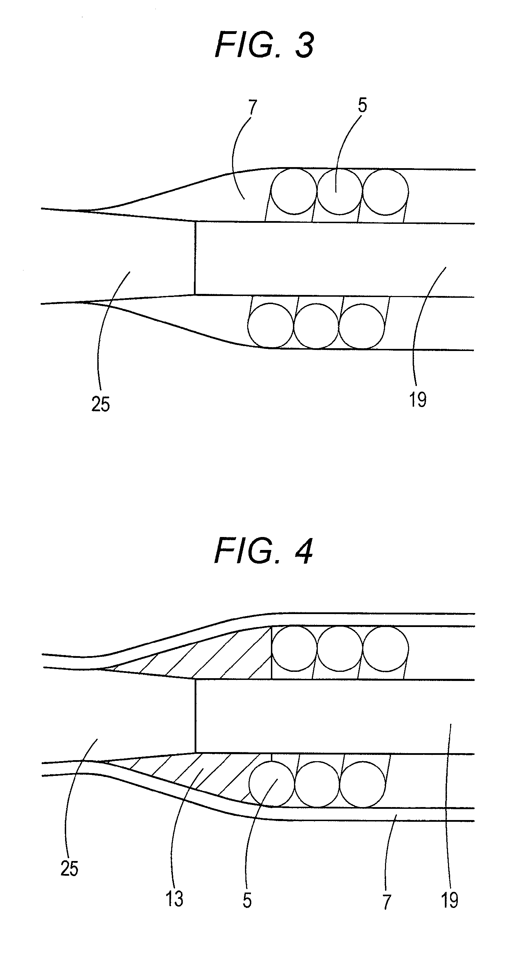

   Fig. 3 illustrates a partially enlarged view of the state of connection between the core shaft and a coiled body in the first embodiment.
Fig. 4
   Fig. 4 illustrates a partially enlarged view of the state of connection between a core shaft and a coiled body in a second embodiment.
Fig. 5

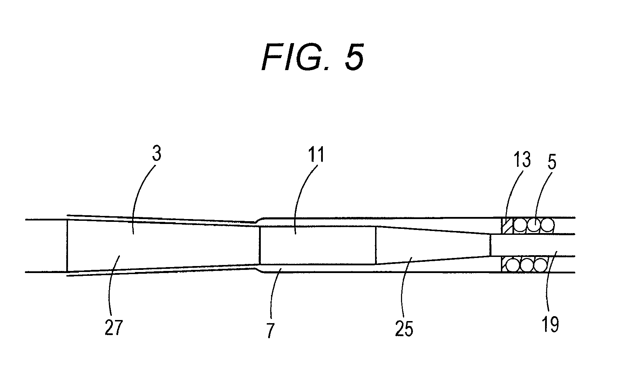

   Fig. 5 illustrates a partially enlarged view of the state of connection between a core shaft and a coiled body in a third embodiment.
Fig. 6

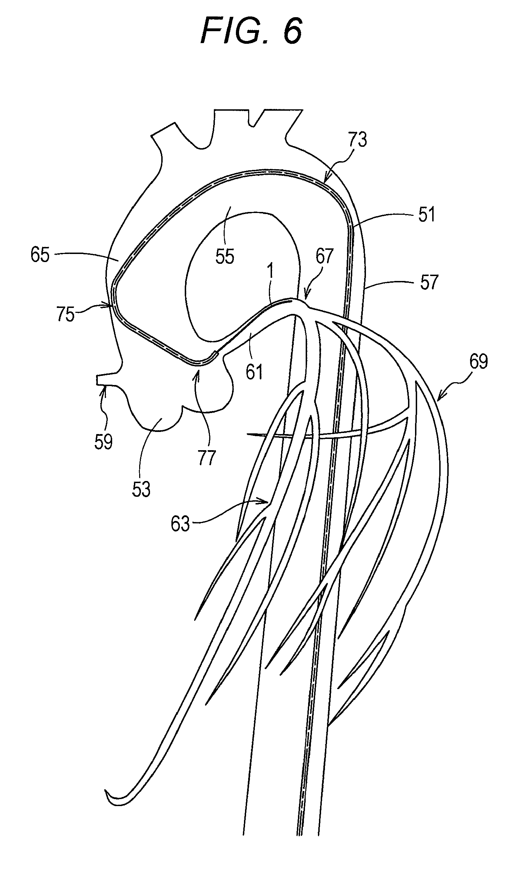

   Fig. 6 illustrates a view of the state of a guiding catheter disposed in an area from a femoral artery to a heart, and a medical guidewire with its tip having reached a left main trunk of a left coronary artery.
Fig. 7

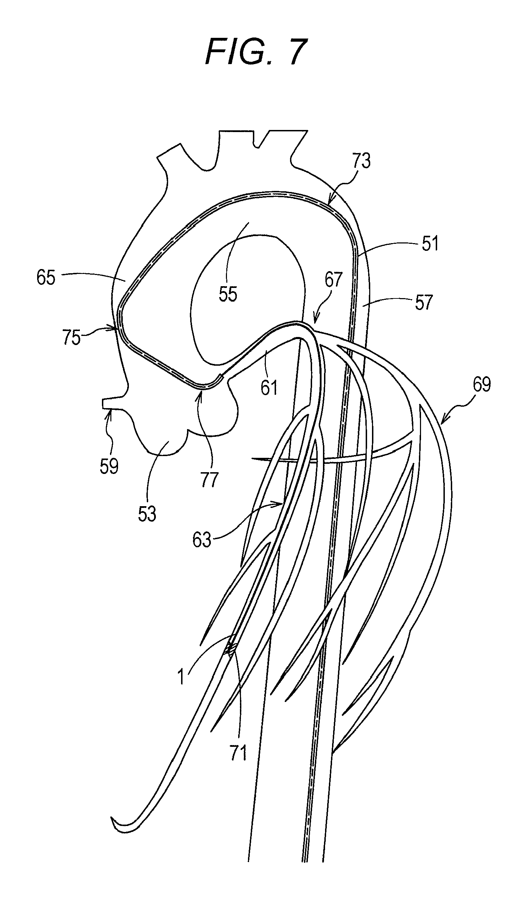

   Fig. 7 illustrates a view of the state of the guiding catheter disposed in the area from the femoral artery to the heart and the medical guidewire with its tip having reached a stenosis part located in a left anterior descending artery of the left coronary artery.
Fig. 8

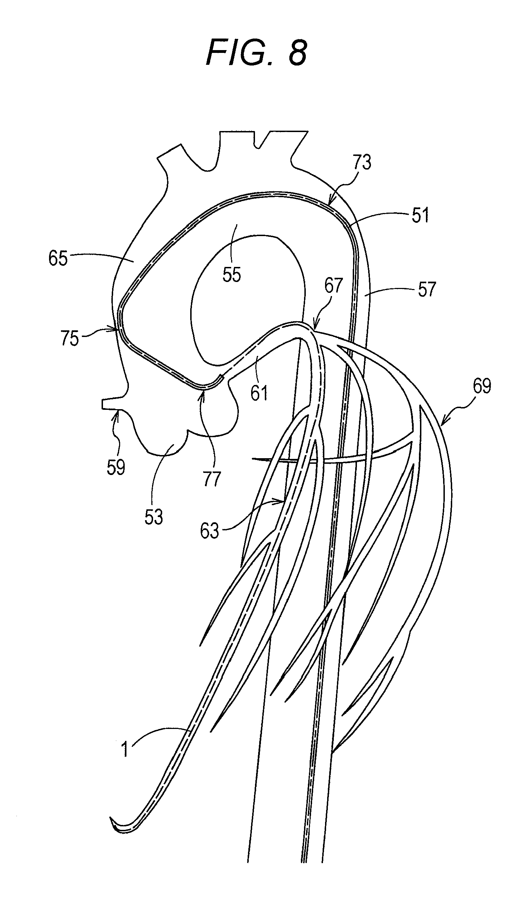

   Fig. 8 illustrates a view of the state of the guiding catheter disposed in the area from the femoral artery to the heart and the medical guidewire with its tip having reached a distal part of the left anterior descending artery.

### Description of Embodiments

A first aspect of the invention is a medical guidewire including a core shaft; and a coiled body that covers at least a tip portion of the core shaft, wherein especially an area between a proximal end portion of the coiled body and the core shaft is formed in streamlined shape.

Further, a second aspect of the invention is the medical guidewire of the first aspect, wherein an outer surface of the proximal end portion of the coiled body and an outer surface of the core shaft are coated with a hydrophilic material, and the streamlined shape is formed by the hydrophilic material.

Further, a third aspect of the invention is the medical guidewire of the first aspect, wherein the proximal end portion of the coiled body and the core shaft are joined to each other by a brazing material, and the streamlined shape is formed by the brazing material.

Further, a fourth aspect of the invention is the medical guidewire of the third aspect, wherein an outer surface of the proximal end portion of the coiled body, an outer surface of the core shaft, and an outer surface of the brazing material are coated with a hydrophilic material.

Further, a fifth aspect of the invention is the medical guidewire of any of the first to fourth aspects, wherein the core shaft has a cylindrical part, with a smaller diameter than that of the coiled body, on the proximal end side of the coiled body, and the cylindrical part is coated with a hydrophilic material.

Further, a sixth aspect of the invention is the medical guidewire of the fifth aspect, wherein a diameter of the cylindrical part including the hydrophilic material is smaller than that of the coiled body.

Further, a seventh aspect of the invention is a medical guidewire, wherein in the medical guidewire of the fifth or sixth aspect, the cylindrical part is provided within a range of 50 to 350 mm from the tip of the core shaft.

According to the first aspect of the invention, the area between the proximal end portion of the coiled body and the core shaft is formed in streamlined shape. It is thus possible to reduce the sliding resistance generated in the case of pulling the medical guidewire inside the catheter, a tubular organ or an intracorporeal tissue. It is thus possible to provide a medical guidewire with favorable manipulability for a physician who performs the operation.

Further, according to the second aspect of the invention, the outer surface of the proximal end portion of the coiled body and the outer surface of the core shaft are coated with the hydrophilic material. Moreover, the streamlined shape is formed by the hydrophilic material. It is thus possible to further reduce the sliding resistance generated in the case of pulling the medical guidewire inside the catheter, the tubular organ or the intracorporeal tissue. It is thus possible to provide a medical guidewire with further favorable manipulability for the physician who performs the operation.

Further, according to the third aspect of the invention, the proximal end portion of the coiled body and the core shaft are joined to each other by the brazing material. Moreover, the streamlined shape is formed by the brazing material. Thereby, in addition to the effect of the invention according to the first aspect, it is possible to easily form the streamlined shape by cutting the brazing material.

Further, according to the fourth aspect of the invention, the outer surface of the proximal end portion of the coiled body, the outer surface of the core shaft, and the outer surface of the brazing material are coated with the hydrophilic material. Thereby, in addition to the effect of the invention according to the third aspect, it is possible to further reduce the sliding resistance generated in the case of pulling the medical guidewire inside the catheter, the tubular organ or the intracorporeal tissue. It is thus possible to provide a medical guidewire with further favorable manipulability for the physician who performs the operation.

Further, according to the fifth aspect of the invention, the core shaft has the cylindrical part, with a smaller diameter than that of the coiled body, on the proximal end side of the coiled body. Moreover, the cylindrical part is coated with the hydrophilic material. Thereby, in addition to the effect of the invention according to any of the first to fourth aspects, it is possible to ensure a space between the guiding catheter and the cylindrical part of the medical guidewire inserted inside the guiding catheter. Furthermore, this core shaft is provided with the cylindrical part having a certain diameter. Therefore, even in the case of the inner surface of the guiding catheter and the outer surface of the guidewire coming into contact with each other when the guiding catheter is curved, the manipulability of the guidewire remains unchanged.

Further, the cylindrical part is coated with the hydrophilic material. It is thus possible to further reduce the sliding resistance between the guiding catheter and the medical guidewire. It is thus possible to provide a medical guidewire with favorable manipulability for the physician who performs the operation.

Further, according to the sixth aspect of the invention, the diameter of the cylindrical part including the hydrophilic material is smaller than that of the coiled body. Therefore, in addition to the effect of the invention according to the fifth aspect, it is possible to provide a medical guidewire with further favorable manipulability for the physician who performs the operation. Further, according to the seventh aspect of the invention, the cylindrical part is provided within the range of 50 to 350 mm from the tip of the core shaft. Therefore, in addition to the effect of the invention according to the fifth or sixth aspect, it is possible to provide the medical guidewire particularly suitable for a curved shape that occurs in the case of applying the guiding catheter to the heart.

Hereinafter, the medical guidewire of the present invention will be described based on preferred embodiments illustrated in the drawings.

### <First Embodiment>

Fig. 1 illustrates an overall view of a medical guidewire according to a first embodiment of the present invention.

It is to be noted that in Fig. 1, a description is given with the left side defined as a "proximal end", and the right side defined as a "tip" for convenience of description.

Further, in Fig. 1, the medical guidewire is reduced in length direction, and illustrated in an overall schematic manner for the sake of easy understanding. An overall size illustrated in Fig. 1 is thus different from an actual size.

In Fig. 1, a medical guidewire 1 is made up of a core shaft 3 and a coiled body 5 that covers a tip portion of the core shaft 3. The tip portion of the core shaft 3 and a tip portion of the coiled body 5 are fixed to each other at an extreme tip portion 9.

A material for the core shaft 3 is not particularly limited. In the present embodiment, stainless steel (SUS304) is used as the material for the core shaft 3. Other than that, a material such as a super elastic alloy like an Ni-Ti alloy, a piano wire, or a tungsten wire may be used.

As a whole, the core shaft 3 has a shape tapering down from the proximal end side toward the tip side. The core shaft 3 includes: a first cylindrical part 21 located in a position with a predetermined distance from the proximal end; a first taper part 27 adjacent to the tip side of the first cylindrical part 21; a second cylindrical part 11 (corresponding to the cylindrical part of the present invention) adjacent to the tip side of the first taper part 27; a second taper part 25 adjacent to the tip side of the second cylindrical part 11; a third cylindrical part 19 adjacent to the tip side of the second taper part 25; a third taper part 29 adjacent to the tip side of the third cylindrical part 19; a fourth cylindrical part 31 adjacent to the tip side of the third taper part 29; a taper press part 33 adjacent to the tip side of the fourth cylindrical part 31; and a cylindrical press part 35 adjacent to the tip side of the taper press part 33.

It is to be noted that the taper press part 33 is one with its side section formed in taper shape by pressing. The cylindrical press part 35 is one with its side section formed in cylindrical shape by pressing.

The second cylindrical part 11 is formed in a range of 50 to 350 mm from the tip of the medical guidewire 1. Herein, the range of 50 to 350 mm is a range corresponding to an area where the medical guidewire 1 is curved by a relatively large amount when used along with a guiding catheter disposed in an area from a femoral artery to a heart.

Herein, an operation of using the medical guidewire 1 and the guiding catheter as medical equipment in combination will be described with reference to the drawings.

Figs. 6 to 8 each illustrate an explanatory drawing of the state of a guiding catheter 51 as medical equipment disposed in the area from the femoral artery to the heart, and the medical guidewire 1. Fig. 6 illustrates a state where the tip of the medical guidewire 1 has reached a left main trunk 61 of a left coronary artery 67. Fig. 7 illustrates a state where the tip of the medical guidewire 1 has reached a stenosis part 71 located in a left anterior descending artery 63 of the left coronary artery 67. Fig. 8 illustrates a state where the tip of the medical guidewire 1 has reached a distal part of the left anterior descending artery 63.

In this operation, first, as illustrated in Fig. 6, a tip portion of the guiding catheter 51 is made to proceed from the femoral artery to a descending aorta 57, an aortic arc 55 and an ascending aorta 65. This tip portion is then fixedly placed in a right coronary artery 59 just before an aortic valve 53 or at one inlet of the left coronary artery 67 (in Fig. 6, the guiding catheter 51 is fixedly placed at the inlet of the left coronary artery 67). The medical guidewire 1 is then inserted inside the fixedly placed guiding catheter 51, and protruded from the tip portion of the guiding catheter 51.

It is to be noted that the left coronary artery 67 is made up of the left main trunk 61, a left circumflex artery 69 and the left anterior descending artery 63. The left main trunk 61 is located upstream of the artery. The left circumflex artery 69 is located downstream of one branch in the left main trunk 61. The left anterior descending artery 63 is located downstream of the other branch in the left main trunk 61.

Herein, for example, in the case where the stenosis part 71 exists in a predetermined position of the left anterior descending artery 63, as illustrated in Fig. 7, the medical guidewire 1 is made to proceed to the position of the stenosis part 71. Thereby, the portion formed with the stenosis part 71 in the artery is treated.

Further, as illustrated in Fig. 8, the guidewire 1 can also be made to proceed to the distal part of the left anterior descending artery 63.

As illustrated in Figs. 6 to 8, the fixedly placed guiding catheter 51 extends almost linearly from the femoral artery, and is curved in a first area 73 from the descending aorta 57 to the aortic arc 55. Moreover, the guiding catheter 51 is curved in a second area 75 from the ascending aorta 65 to the left coronary artery 67, with a smaller radius of curvature, and is curved in a third area 77 in the vicinity of the inlet of the left coronary artery 67, with an even smaller radius of curvature.

As described above, the second cylindrical part 11 is formed in the range of 50 to 350 mm from the tip of the medical guidewire 1. This range corresponds to the third area 77, the second area 75, and the first area 73.

In such a manner, in the medical guidewire 1, the range of 50 to 350 mm from the tip is formed in cylindrical shape as the second cylindrical part 11. This is for making uniform the sliding resistance between the guiding catheter 51 and the medical guidewire 1 in the third area 77, the second area 75 and the first area 73.

In contrast, when the second cylindrical part 11 is formed not in cylindrical shape but in, for example, tapered shape or conical shape, the sliding resistance may change in the third area 77, the second area 75 or the first area 73. Hence the manipulability of the medical guidewire 1 may decrease during an operation.

Further, the radii of curvature of the medical guidewire 1 in the second area 75 and the third area 77 are smaller than the radius of curvature in the first area 73. Hence, the effect can also be obtained even when the formation range of the second cylindrical part 11 is restricted only to a range corresponding to the second area 75 and the third area 77. In that case, the second cylindrical part 11 may be formed in a range of 50 to 250 mm from the tip of the medical guidewire 1.

It is to be noted that in the present embodiment, the second cylindrical part 11 of the medical guidewire 1 is formed based on the shape of the guiding catheter 51 disposed in the area from the femoral artery to the heart. However, a curved position varies depending on the site in the body where the guiding catheter 51 is used. Therefore, in the case of using the guiding catheter 51 in another site in the body, the second cylindrical part 11 of the medical guidewire 1 is preferably formed in accordance with a curved position of that site in the body.

Moreover, in the case of using the medical guidewire 1 and a medical equipment other than the guiding catheter in combination, the second cylindrical part 11 is preferably formed in accordance with a curved shape of the medical equipment.

However, in any case, common points are to form the second cylindrical part 11 in cylindrical shape, to form the second cylindrical part 11 on the side closer to the proximal end than the coiled body 5, and to form the second cylindrical part 11 with a smaller outer diameter than that of the coiled body 5.

Forming the second cylindrical part 11 on the proximal end side of the coiled body 5, with the smaller outer diameter than that of the coiled body 5, exerts the following effect.

That is, when the coiled body 5 including the extreme tip portion 9 of the medical guidewire 1 is inserted inside the body, the coiled body 5 passes through the inside of the body. Hence, in the body, a cavity corresponding to the outer diameter of the coiled body 5 is formed on the proximal end side of the coiled body 5. The second cylindrical part 11 is formed, with a smaller outer diameter than that of the coiled body 5, on the proximal end side of the coiled body 5. For this reason, a space is generated between the second cylindrical part 11 and an inner wall of the cavity formed by passage of the coiled body 5.

Generation of this space can reduce the sliding resistance between the second cylindrical part 11 and the inner wall of the cavity.

Further, as illustrated using Figs. 6 to 8, when the guiding catheter 51 is curved, or even when the medical guidewire 1 itself is solely curved in the body, it is possible to reduce the sliding resistance between the second cylindrical part 11 and the cavity formed by passage of the coiled body 5.

A material for the coiled body 5 is not particularly limited. In the present embodiment, stainless steel (SUS304) is used as the material for the coiled body 5. Other than that, similarly to the core shaft 3, a material such as a super elastic alloy like an Ni-Ti alloy, a piano wire, or a tungsten wire may be used.

The coiled body 5 is wound in coiled shape around the tip portion of the core shaft 3. The coiled body 5 is fixed by brazing to the tip portion of the core shaft 3 in a plurality of places, including a coil-tip brazed portion 15 continued to the extreme tip portion 9, a plurality of coil-middle brazed portions that are located on the proximal end side of the coil-tip brazed portion 15 and include a coil-middle brazed portion 17, and a coil-base-end brazed portion 13 located at the proximal end of the coiled body 5.

It should be noted that in Fig. 1, only one coil-middle brazed portion 17 is illustrated, and the other coil-middle brazed portions are omitted.

In the vicinity of the extreme tip portion 9, the coiled body 5 is wound so as to generate a space between adjacent wires of the coiled body 5. On the other hand, on the proximal end side from the coil-middle brazed portion 17 that is adjacent to the proximal end side of the coil-tip brazed portion 15, the coiled body 5 is wound such that adjacent wires of the coiled body 5 are in contact with each other.

It is to be noted that diameters of the wires of the coiled body 5 in the present embodiment are uniform. However, the diameters of the wires of the coiled body 5 may be decreased gradually from the proximal end toward the tip of the coiled body 5. Further, the diameters of the wires of the coiled body 5 on the side closer to the tip than the coil-middle brazed portion 17 may be made smaller than those of the other wires of the coiled body 5.

Decreasing the diameters of the wires of the coiled body 5 gradually from the proximal end toward the tip of the coiled body 5 can gradually enhance the flexibility of the tip portion of the medical guidewire 1. This is effective in the case of curving the entire coiled body 5.

Meanwhile, making smaller the diameters of the wires of the coiled body 5, provided on the side closer to the tip than the coil-middle brazed portion 17, than those of the other wires of the coiled body 5 can enhance the flexibility of a portion on the side closer to the tip than the coil-middle brazed portion 17. This is effective in the case of curving the area on the side closer to the tip than the coil-middle brazed portion 17, with a relatively small radius of curvature.

Further, the outer surfaces of the medical guidewire 1 from the extreme tip portion 9 to the coiled body 5 and the second cylindrical part 11 are coated with a hydrophilic material 7.

Examples of the hydrophilic material may include a cellulose-based polymer, a polyethylene oxide-based polymer, a maleic anhydride-based polymer (e.g., maleic anhydride copolymer such as methyl vinyl ether-maleic anhydride copolymer), an acrylamide-based polymer (e.g., polyacrylamide, polyglycidyl methacrylate-dimethylacrylamide block copolymer), water-soluble nylon, polyvinyl alcohol, polyvinylpyrrolidone, and hyaluronate.

It is to be noted that the hydrophilic material 7 in the present embodiment is hyaluronate.

The hydrophilic material 7 coated on the medical guidewire 1 reduces the sliding resistance of the medical guidewire 1 inside the catheter, the tubular organ or the intracorporeal tissue.

By coating the second cylindrical part 11, with a smaller outer diameter than that of the coiled body 5, with the hydrophilic material 7 in the present embodiment, it is possible to significantly reduce the sliding resistance between the guiding catheter and the medical guidewire 1.

By applying the hydrophilic material 7 onto the outer surfaces of the medical guidewire 1 from the extreme tip portion 9 to the coiled body 5 and the second cylindrical part 11 such that the thickness is made uniform, it is possible to sufficiently reduce the sliding resistance.

It should be noted that the number of sliding of the medical guidewire 1 and the guiding catheter increases when a long period of time is required for the physician to perform the operation. In the present embodiment, consideration is also given in that respect.

Fig. 2 illustrates a partially enlarged view of the second cylindrical part of the core shaft in the present embodiment.

In Fig. 2, the hydrophilic material 7 coated on the second cylindrical part 11 is formed with a larger thickness than that of the hydrophilic material 7 coated on the coiled body 5.

That is, the hydrophilic material 7 is formed on the surface of the core shaft 3 such that a distance from the outer surface of the second cylindrical part 11 to the outer surface of the hydrophilic material 7 is longer than a distance from the outer surface of the coiled body 5 to the outer surface of the hydrophilic material 7.

Further, the hydrophilic material 7 coated on the second cylindrical part 11 is formed with a larger thickness than those of the hydrophilic materials 7 coated on the first taper part 27 and the second taper part 25 which are adjacent to the second cylindrical part 11.

Therefore, as illustrated in Figs. 6 to 8, even when the medical guidewire 1 is slid for a long period of time with the guiding catheter curved, the sliding resistance in the second cylindrical part 11 does not increase.

Further, the diameter of the second cylindrical part 11 including the hydrophilic material 7 is desirably smaller than that of the coiled body 5 including the hydrophilic material 7.

Making smaller the diameter of the second cylindrical part 11 including the hydrophilic material 7 than that of the coiled body 5 including the hydrophilic material 7 can generate a space between a cavity, formed by passage of the coiled body 5 through the inside of the guiding catheter, the tubular organ or the intracorporeal tissue, and the second cylindrical part located on the proximal end side of the coiled body 5. Thereby, even when the medical guidewire 1 is slid for a long period of time with the guiding catheter curved, the sliding resistance between the guiding catheter and the medical guidewire 1 can be kept low.

Meanwhile, it is also preferable to reduce the sliding resistance of the medical guidewire 1 not only in the case of pushing the medical guidewire 1 but also in the case of pulling the medical guidewire 1. In the present invention, consideration is also given in that respect.

Fig. 3 illustrates a partially enlarged view of the state of connection between the core shaft and the coiled body in the present embodiment.

As illustrated in Fig. 3, the hydrophilic material 7 is applied so as to form a streamlined shape in the area from the proximal end portion of the coiled body 5 to the second taper part 25. It is thus possible to reduce the sliding resistance of the medical guidewire 1 at the time of pulling the medical guidewire 1 inside the guiding catheter, the tubular organ or the intracorporeal tissue.

In the present embodiment, the hydrophilic material 7 is applied so as to form the streamlined shape in the area from the proximal end portion of the coiled body 5 to the second taper part 25. However, this hydrophilic material 7 may be applied so as to form the streamlined shape in the area from the proximal end portion of the coiled body 5 to the second cylindrical part 11. It is thus possible to reduce a depressed portion of the second taper part 25. It is further possible to reduce the sliding resistance of the medical guidewire 1 at the time of pulling the medical guidewire 1 inside the guiding catheter, the tubular organ or the intracorporeal tissue.

It is to be noted that also in this case, the hydrophilic material 7 applied onto the second cylindrical part 11 is preferably formed with a larger thickness than those of the hydrophilic materials 7 applied onto the coiled body 5, the first taper part 27 and the second taper part 25.

In that case, it is possible to more favorably inhibit the increase in sliding resistance in the second cylindrical part 11 in the case of sliding the medical guidewire 1 for a long period of time with the guiding catheter curved.

### <Second Embodiment>

Next, a second embodiment of the medical guidewire of the present invention will be described.

Fig. 4 illustrates a partially enlarged view of the state of connection between a core shaft and a coiled body in the second embodiment.

In Fig. 4, the coil-base-end brazed portion 13 is made of a brazing material in streamlined shape which is formed in the area from the proximal end portion of the coiled body 5 to the second taper part 25. The hydrophilic material 7 is applied with a uniform thickness onto the range from the coiled body 5 to the coil-base-end brazed portion 13 and the second taper part 25.

In this case, it is possible to easily form the streamlined shape by cutting the brazing material. It is thus possible to easily manufacture a medical guidewire reduced in sliding resistance.

Also by means of this medical guidewire 1 of the second embodiment, it is possible to reduce the sliding resistance of the medical guidewire 1 at the time of pulling the medical guidewire 1 inside the guiding catheter, the tubular organ or the intracorporeal tissue.

In the second embodiment, in the area from the proximal end portion of the coiled body 5 to the second taper part 25, the brazed portion 13 is formed by use of the brazing material so as to form the streamlined shape. However, the area from the proximal end portion of the coiled body 5 to the second cylindrical part 11 may be formed in the streamlined shape by use of this brazing material. It is thus possible, as in the first embodiment, to reduce the depressed portion of the second taper part 25. It is further possible to reduce the sliding resistance of the medical guidewire 1 at the time of pulling the medical guidewire 1 inside the guiding catheter, the tubular organ or the intracorporeal tissue.

In this case, the hydrophilic material 7 is applied with a uniform thickness onto the area from the coiled body 5 to the coil-base-end brazed portion 13 and the second cylindrical part 11.

It is to be noted that also in this case, the hydrophilic material 7 applied onto the second cylindrical part 11 may be formed with a larger thickness than those of the hydrophilic materials 7 applied onto the coiled body 5, the first taper part 27 and the second taper part 25.

In that case, it is possible to more favorably inhibit the increase in sliding resistance in the second cylindrical part 11 in the case of sliding the medical guidewire 1 for a long period of time with the guiding catheter curved.

### <Third Embodiment>

Next, a third embodiment of the medical guidewire of the present invention will be described.

Fig. 5 illustrates a partially enlarged view of the state of connection between a core shaft and a coiled body in the third embodiment.

In the third embodiment, as illustrated in Fig. 5, the hydrophilic material 7 is applied so as to form a cylindrical shape (or linear shape) in a middle area from the proximal end portion of the coiled body 5 to the second cylindrical part 11. Further, the hydrophilic material 7 is applied so as to form a curved shape in a connecting section between this middle area and the proximal end portion of the coiled body 5, and applied so as to form a curved shape in a connecting section between the middle area and the second cylindrical part. It is thus possible to further reduce the sliding resistance of the medical guidewire 1 at the time of pulling the medical guidewire 1 inside the guiding catheter, the tubular organ or the intracorporeal tissue.

While the invention has been shown and described in detail, the foregoing description is in all aspects illustrative and not restrictive. It is therefore understood that numerous modifications and variations can be devised without departing from the spirit and scope of the invention.

Reference Signs List
- 1: medical guidewire
- 3: core shaft
- 5: coiled body
- 7: hydrophilic material
- 9: extreme tip portion
- 11: second cylindrical part
- 13: coil-base-end brazed portion
- 15: coil-tip brazed portion
- 17: coil-middle brazed portion
- 19: third cylindrical part
- 21: first cylindrical part
- 25: second taper part
- 27: first taper part
- 29: third taper part
- 31: fourth cylindrical part
- 33: taper press part
- 35: cylindrical press part
- 53: aortic valve
- 55: aortic arc
- 57: descending aorta
- 59: right coronary artery
- 61: left main trunk
- 63: left anterior descending artery
- 65: ascending aorta
- 67: left coronary artery
- 69: left circumflex artery
- 71: stenosis part

## Claims

1. A medical guidewire comprising:
a core shaft; and
a coiled body that covers at least a tip portion of the core shaft,
wherein an area between a proximal end portion of the coiled body and the core shaft is formed in streamlined shape.

2. The medical guidewire according to claim 1, wherein an outer surface of the proximal end portion of the coiled body and an outer surface of the core shaft are coated with a hydrophilic material, and
the streamlined shape is formed by the hydrophilic material.

3. The medical guidewire according to claim 1, wherein the proximal end portion of the coiled body and the core shaft are joined to each other by a brazing material, and
the streamlined shape is formed by the brazing material.

4. The medical guidewire according to claim 3, wherein an outer surface of the proximal end portion of the coiled body, an outer surface of the core shaft, and an outer surface of the brazing material are coated with a hydrophilic material.

5. The medical guidewire according to any one of claims 1 to 4, wherein the core shaft has a cylindrical part, with a smaller diameter than that of the coiled body, on the proximal end side of the coiled body, and
the cylindrical part is coated with a hydrophilic material.

6. The medical guidewire according to claim 5, wherein a diameter of the cylindrical part including the hydrophilic material is smaller than that of the coiled body.

7. The medical guidewire according to claim 5 or 6, wherein the cylindrical part is provided within a range of 50 to 350 mm from the tip of the core shaft.
